# EUROPEAN PATENT SPECIFICATION

(11) **EP 2 405 885 B1**
(45) Date of publication and mention of the grant of the patent: **24.10.2018**
(21) Application number: 10751412.7
(22) Date of filing: 11.03.2010
(51) Int. Cl.: A61K 8/81, A61Q 19/00, A61Q 17/04

(54) **THICKENING ADDITIVE COMPOSITIONS FOR SUNSCREEN FORMULATIONS**
VERDICKENDE ZUSATZSTOFFE FÜR SONNENSCHUTZ ZUSAMMENSETZUNGEN
COMPOSITIONS D'ADDITIF EPAISSISSANT POUR FORMULATIONS SOLAIRES

(30) Priority: 11.03.2009 US 159284 P; 08.04.2009 US 167727 P
(43) Date of publication of application: 18.01.2012
(73) Proprietor: ISP Investments LLC, Wilmington, DE 19805 (US)
(72) Inventor: FARES, Hani, Somerset NJ 08873 (US); BARRETT, Chris, Oakland NJ 07436 (US); SHIH, Jenn, Paramus NJ 07652 (US); BOTSCHKA, Ellen, Riverdale NJ 07457 (US); ROSS, Tracey, Hewitt NJ 07421 (US)
(74) Representative: Harrison, Susan Joan
(86) International application number: PCT/US2010/026973
(87) International publication number: WO 2010/105050

(56) References cited:
- WO-A1-98/52536
- WO-A1-2006/128255
- US-A- 5 073 614
- US-A- 5 139 770
- US-A- 5 139 770
- US-A- 5 663 258
- US-A- 5 830 447
- US-A1- 2007 264 204
- US-A1- 2008 057 017
- US-A1- 2008 113 895

## Description

This application claims the benefit of U.S. Provisional App. Ser. No. 61/159,284 filed March 11, 2009 and U.S. Provisional App. Ser. No. 61/167,727 filed April 8, 2009.

### FIELD

The present invention relates to a sunscreen composition comprising a thickening additive composition comprising: (A) one or more substantially anhydrous solvent systems; and (B) a thickening agent, wherein the thickening agent comprising a strongly swellable, lightly to moderately crosslinked polyvinylpyrrolidone. The polyvinylpyrrolidone typically has an aqueous gel volume of about 15 to 150 mL/gm of polymer and/or a Brookfield viscosity in 5% aqueous solution of at least 10,000 cP at 25°C. The additive compositions could be added to the base composition to make personal care products for skin care, oral care, sun care, hair care and toiletries. More particularly, personal care compositions that are difficult to thicken include those that are substantially anhydrous or have low pH. The additive compositions described herein are intended to maintain desired viscosity, rheology and stability characteristics of the base composition.

### BACKGROUND

The personal care category consists of a broad array of products that are fabricated in varied formulation types such as foundations, concealers, lipsticks, lotions, suncare, sunless tanning, face creams, liquid soaps, mascaras, and other color cosmetics and delivery systems. These products provide a wide range of perceived efficacies to the consumer, from "light" daily care products to therapeutic ointments and creams.

Creams, lotions and gels are frequently used topically for cosmetic or pharmaceutical use. Whether used to deliver a drug or as a skin moisturizer, the consistency and feel of the composition are important to the commercial success of the product. Stabilized compositions are important in formulating such products.

Aqueous cosmetic compositions often require thickeners. Thickeners which have been previously used in formulating creams, lotions and gels include several commercially available acrylic acid polymers and polyacrylamides. However, to obtain a target viscosity the manufacturers of these thickeners consistently recommend that the thickener be dispersed in water and then neutralized. Thus, in aqueous formulation systems acrylic acid polymers and polyacrylamides have been found quite useful and acceptable.

Unlike their aqueous counterparts, anhydrous systems are especially difficult to thicken and also may present formulation stability problems. Examples of various anhydrous systems that are difficult to thicken and stabilize include alcohols, esters, glycols, acids, oils, and hydrocarbons.

Many thickeners, also known as rheological modifiers, are characterized as natural, modified-natural and synthetic. Natural rheological modifiers include guar gum, pectin, casein, carrageenan, xanthan gum and alginates. Modified rheological modifiers include chemically modified celluloses, most particularly methylcellulose, hydroxyethylcellulose, hydroxypropylcellulose, and carboxymethylcellulose. Synthetic thickeners include derivatives of the former categories, and also polyvinyl alcohols, polyacrylamides, polyvinylpyrrolidones, various polyethers, their copolymers as well as polyacrylic acids and their salts.

U.S. Patent No. 5,422,112 discloses a thickener system including a combination of xanthan gum, magnesium aluminum silicate and polyacrylamide. The compositions are said to be particularly effective at low pH used especially for thickening alpha-hydroxy carboxylic acids and salts thereof.

Similarly, U.S. Patent No. 5,874,095 claims an enhanced skin penetration system comprising a nonionic polyacrylamide of high molecular weight, for improved topical delivery of drugs at low pH.

Further, U.S. Patent No. 6,774,100 reports an anhydrous composition having a viscosity greater than 1000 centipoise (cP) maintained with a thickening agent selected from the group consisting of acrylic acid polymers and polyacrylamides.

Some acrylic acid thickeners are described in U.S. Patent Nos. 2,883,351; 2,956,046; 3,035,004; and 3,436,378.

Polyvinylpyrrolidone and its salts and esters being used as rheology modifier or as thickener in various personal care products are described in U.S. Patent Nos. 6,436,380; 6,197,281; 6,333,039; 6,685,952; and 7,108,860.

U.S. Patent Application Publication No. 2003/0118620 discloses a thickening system for cosmetic compositions of low pH, comprising polysaccharide and taurate copolymer.

Nonetheless, acrylic, polyacrylamide, and silicone elastomers are widely acceptable thickener systems for aqueous system; there still exists a need for thickening a substantially anhydrous composition, which provides desired viscosity and stability.

The present application is directed to personal care composition with improved thickening characteristics and associated stability. In accordance with certain aspects, one or more of the following benefits may be realized:
- a thickening system for wide range of anhydrous compositions including gels, creams, lotions, solutions, emulsions etc;
- a system which is easy to use;
- thickening to substantially anhydrous sunscreens;
- a thickening additive composition which is compatible at a wide pH range, particularly at lower pH;
- a thickening additive composition which comprises dihydroxyacetone, surfactants, salts and electrolytes;
- a thickening additive composition which is stable or maintains viscosity at high shear.

Accordingly, certain embodiments described herein may provide a thickening additive system and thus thickened personal care composition of sufficient aesthetic viscosity and skin feel.

### SUMMARY

The present invention is a sunscreen composition comprising a thickening additive composition wherein the thickening additive composition comprises (A) one or more substantially anhydrous solvent systems comprising 15% or less water, the solvent including one or more of esters, glycols, acids, oils, emulsions and hydrocarbons; and (B) a thickening agent, wherein the thickening agent comprises a strongly swellable, crosslinked polyvinylpyrrolidone having an aqueous gel volume of 15 to 150 mL/gm of polymer and a Brookfield viscosity in 5% aqueous solution of at least 10,000 cP at 25°C, measured using a Brookfield viscometer; wherein said thickening agent is from 1% to 6% by weight of said thickening additive composition.

The substantially anhydrous solvent system may further comprise other chemicals selected from the group consisting of salts, electrolytes, acids, bases, surfactants, cations, di-hydroxyacetone, and combinations thereof.

The additive composition may be added to a base composition to make personal care products, such as products for skin care, hair care, sun care, oral care and toiletries.

In accordance with another aspect of the invention, the sunscreen composition further comprises: (i) a personal care active and (ii) a cosmetically or dermatologically-acceptable carrier.

Examples of personal care compositions include shampoos, body wash products, shaving cream, hand soap, bubble bath, bath gel, after-shave lotions, creams, moisturizers, sunscreens, liquid soaps, color cosmetics, acid peels, perms, hair color, sunless tanning and conditioners. The cosmetically or pharmaceutically acceptable carrier may be selected from the group consisting of a cream, a lotion, an emulsion, an oil, a spray, a gel, an aerosol, an aqueous or hydroalcoholic solution, a suspension, a powder, a serum, an ointment, a shampoo, a gel, a paste and a mousse.

Personal care compositions can be produced wherein the composition exhibits a viscosity of at least 7000 cP at 3% concentration of thickening additive composition, and a viscosity of at least 30,000 cP at 5% concentration.

The composition may further comprise at least one adjuvant such as a diluent, a lubricant, a surfactant, a flowing agent, an emollient, humectants, an antioxidant, a preservative, an antimicrobial agent, an antibiotic and an emulsifier.

The composition can be used in a great number of treatments, notably cosmetic and dermatological. They can be fabricated in the form of cosmetic compositions used for skin, lips and/or hair treatment, protection, care and make-up removal and/or cleaning, as well as for make-up applications on skin, lips, eye lashes and/or the body. The personal care composition may be formulated in the form of liquid, gel, semi solid, or solid.

Thickening additive compositions are effective in imparting improved viscosity, stability characteristics to the personal care composition and also maintain viscosity after exposure to high shear.

The thickening additive composition may increase and maintain the viscosity of compositions comprising acids. More particularly, the disclosed thickening additive composition may provide thickening to low pH personal care compositions.

Compositions disclosed herein may also provide stability to an anhydrous sun screen system. More particularly, the compositions may thicken the anhydrous sunscreen system and provide water resistance in sunscreen formulations.

### DETAILED DESCRIPTION

The disclosure relates to improved thickening systems for cosmetic compositions, particularly to substantially anhydrous compositions, such as anhydrous creams, lotions and gels useful in the personal care industries. More particularly, this disclosure relates to a thickening additive composition comprising a substantially anhydrous solvent system, and a thickening agent. The additive composition enables the formulator to thicken non-aqueous systems creating anhydrous products and is applicable to a wide range of materials including esters, glycols, acids, mineral oil, vegetable oils, sunscreens and hydrocarbons; used either alone or in combination.

The present invention involves a strongly swellable, moderately crosslinked polyvinylpyrrolidone constitute a thickening system which can easily be utilized for formulating various personal care products. More particularly, the thickening systems are useful in personal care compositions with low pH or high acid content; sunscreens; emulsions; and oils. The resulting products showed improved viscosity and thickening properties, without stability issues.

The term "thickening additive composition" as used herein refers to a composition capable of providing thickness and rheological properties, particularly to substantially anhydrous solvent systems.

The term "personal care composition" as used herein refers to composition whose function is to maintain, restore or improve the appearance of skin and hair. Specific examples of personal care compositions include, but are not limited to, compositions with low pH or high acid content; sunscreens; emulsions; and oils.

Accordingly, a first element of the thickening additive composition according to the present invention is the thickening agent comprising strongly swellable, lightly to moderately crosslinked polyvinylpyrrolidone as described in commonly assigned U.S. Patent Nos. 5,312,619 and 5,139,770. The term "strongly swellable, lightly to moderately crosslinked PVP", unless otherwise noted, specifically refers to polymer essentially consisting of lightly- to moderately-crosslinked poly(*N*-vinyl-2-pyrrolidone) having at least one of the following characteristics: (1) an aqueous swelling parameter defined by its gel volume from about 15 mL/g to about 300 mL/g, more particularly from about 15 mL/g to about 250 mL/g, and in other cases from about 15 mL/g to about 150 mL/g, or (2) a Brookfield viscosity of 5% crosslinked PVP in a liquid carrier comprising water at 25°C of at least 2,000 cP, more particularly of at least about 5,000 cP, and in certain cases of at least about 10,000 cP. Disclosure for these parameter ranges is provided in U.S. patent 5,073,614 and in Shih, J.S., *et al.* (1995). Synthesis methods for the crosslinked PVP are disclosed in a number of references, including U.S. patents 5,073,614; 5,654,385; and 6,177,068. It is appreciated by a polymer scientist skilled in the art that the method of synthesis is immaterial, inasmuch as the produced polymer achieves at least one of the above defined parameters.

For example, U.S. patent '614 discloses different crosslinkers and crosslinker amounts that yield crosslinked PVP suitable for the present invention. The effect of crosslinker amount on swell volume and viscosity is graphically presented in Shih, J.S., *et al.* (1995). Thus, the crosslinked PVP may be produced by the precipitation polymerization method of the '614 patent, by the hydrogel method described in the '385 patent, or by the non-aqueous, heterogeneous polymerization method of the '068 patent. Certainly, other techniques are contemplated to synthesize this polymer, provided the product meets the aqueous swelling parameter and Brookfield viscosity requirements.

Final product viscosities may slightly vary for compositions containing crosslinked PVP made by these different methods. Nonetheless, these variations are within the scope of the invention, as the crosslinked PVPs thicken low pH compositions.

Unless otherwise specified, "strongly swellable, lightly to moderately crosslinked PVP" does not refer to swellable but water-insoluble crosslinked PVP, such as the type sold into commercial trade under the trade name Polyclar® by International Specialty Products, which differs from the above described crosslinked PVP.

Commercially available examples of strongly swellable, lightly to moderately crosslinked PVP include, but are not limited to, ACP-1120, ACP-1179, and ACP-1180, available from International Specialty Products (Wayne, NJ).

As known to those skilled in the art, the precise amount of thickeners can vary depending upon the consistency and thickness of the composition which is desired. With reference to present invention, thickening agent/thickeners/rheology modifiers are used in amounts from 1% to 6% by weight of the composition.

In accordance with certain embodiments, the compositions have a viscosity greater than about 7000 centipoise (cP) at 3% concentration when measured using a Brookfield viscometer at room temperature. It should be understood that all viscosities referred to herein are measured in this manner. In accordance with particular embodiments, the composition has a viscosity greater than 30,000 cP at 5% concentration. In particularly useful embodiments, the composition has a viscosity in the range of from about 1000 to about 100,000 centipoise. In other embodiments, the compositions have a viscosity in the range of about 10,000 cP to about 100,000 cP.

The substantially anhydrous solvents useful in the present compositions include esters, glycols, acids, oils, emulsions, and hydrocarbons; used either alone or in combination.

Useful glycols include, but are not limited to, propylene glycol, butylene glycol, diethylene glycol, dipropylene glycol, polyethylene glycol, glycerin, and mixtures thereof.

Useful oils include, but are not limited to, castor bean oil, castor oil, rapeseed oil, soybean oil, palm kernel oil, babassu kernel oil, coconut oil, and mixtures thereof.

Useful acids include, but are not limited to, alpha and beta hydroxy acid, alpha hydroxyethanoic acid, alpha hydroxyoctanoic acid alpha hydroxycaprylic acid, ascorbic acid, adipic acid, citric acid, caprylic acid, capric acid, glycolic acid, lactic acid, lauric acid, malic acid, mixed fruit acids, myristic acid, palmitic acid, salicylic acid, stearic acid, linoleic acid, linolenic acid, ricinoleic acid, oleic acid, elaidic acid, erucic acid, and mixtures thereof.

The substantially anhydrous solvent system may further comprise other chemicals such as salts, electrolytes, acids, bases, surfactants, cations, and di-hydroxyacetone.

The present application discloses compositions containing a "substantially anhydrous solvent system" together with a thickening agent, defined hereafter. The composition constitutes an additive composition which can easily be added to a base composition to make personal care products such as those for skin care, hair care, sun care, oral care and toiletries. The blended inventive product results in a thickening composition with equal or improved viscosity and thickening properties.

Within the context of the present invention, the resulting personal care composition is an anhydrous preparation, which will be understood by those of skill in the art, to mean that water has not been added as a component. However, water may be present in the composition via its presence in the formulation components and absorption from the atmosphere.

The compositions which include chemicals such as salts, electrolytes, acids, bases, surfactants, cations, and di-hydroxyacetone may have water, if any, in less than 15%, more particularly less than 10% and even more particularly less than 5%.

Also described is a personal care composition comprising a personal care active; (ii) a cosmetically or dermatologically-acceptable carrier; and (iii) thickening additive composition comprising (A) one or more substantially anhydrous solvent systems; and (B) a thickening agent, wherein the thickening agent comprises a strongly swellable, moderately crosslinked polyvinylpyrrolidone having an aqueous gel volume of about 15 to 150 mL/gm of polymer and a Brookfield viscosity in 5% aqueous solution of at least 10,000 cP at 25°C.

Examples of personal care compositions include, but are not limited to, shampoo, body wash products, shaving cream, hand soap, bubble bath, bath gel, after-shave lotions, creams, moisturizers, sunscreens, liquid soaps, color cosmetics, acid peels, perms, hair color, sunless tanning and conditioners.

In most cases, the personal care products comprise a personal care active ingredient which provides benefit to the user's body, e.g., to the hair or skin. Such materials are in general well-known to those persons of ordinary skill in the relevant personal care composition art, and may include moisturizing agents, antiperspirants, anti-bacterials, sunscreen agents, insect repellents, cleaning agents, hair conditioning agents, hair styling agents, anti-dandruff agents, hair growth promoters, hair dyes and pigments, anesthetics, lubricants, spermicides, soaps and perfumes.

The active personal care ingredients (and other ingredients of the personal care compositions as described below) can be categorized by the benefit they provide or by their postulated mode of action. However, it is to be understood that the active personal care ingredients (and other ingredients) useful herein can in some instances provide more than one benefit or operate via more than one mode of action. Therefore, classifications herein are made for the sake of convenience and are not intended to limit the active to that particular application or applications listed.

Examples of substances that may suitably be included in the personal care products as active personal care ingredients include the following:
(1) perfumes and fragrances, which give rise to an olfactory response in the form of a fragrance, and deodorant perfumes which, in addition to providing a fragrance response, can also reduce body malodor;
(2) skin coolants, such as menthol, menthyl acetate, menthyl pyrrolidone carboxylate, N-ethyl-p-menthane-3-carboxamide and other derivatives of menthol, which give rise to a tactile response in the form of a cooling sensation on the skin;
(3) emollients, such as isopropylmyristate, silicone oils, mineral oils and vegetable oils, which give rise to a tactile response in the form of an increase in skin lubricity;
(4) deodorants other than perfumes, whose function is to reduce the level of or eliminate micro flora at the skin surface, especially those responsible for the development of body malodor, including precursors of deodorants;
(5) antiperspirant actives, whose function is to reduce or eliminate the appearance of perspiration at the skin surface;
(6) moisturizing agents, that keep the skin moist by either adding moisture or preventing from evaporating from the skin;
(7) cleansing agents, that remove dirt and oil from the skin;
(8) sunscreen active ingredients that protect the skin and hair from UV and other harmful light rays from the sun;
(9) hair treatment agents that condition hair, clean hair, detangle hair, act as styling agents, anti-dandruff agents, hair growth promoters, hair dyes and pigments, hair perfumes, hair relaxers, hair bleaching agents, hair moisturizers, hair oil treatment agents and antifrizzing agents;
(10) oral care agents, that clean, whiten, deodorize and protect the teeth and gum;
(11) denture adhesives, that provide adhesion properties to dentures;
(12) beauty aids, such as powders, pigments and colorants; and
(13) medicinal agents.

Further examples of skin benefit agents include abrasives; absorbents; aesthetic components such as opacifying agents and pearlescent aids such as ethylene glycol distearate and TiO₂ coated mica; essential oils; skin sensates; cosmetic and drug astringents such as clove oil, menthol, camphor, eucalyptus oil, eugenol, menthyl lactate and witch hazel distillate; anti-acne agents such as resorcinol, sulfur, salicylic acid, benzoyl peroxide, erythromycin and zinc; anti-caking agents; antimicrobial agents such as iodopropyl butylcarbamate; antioxidants; cosmetic biocides; external analgesics; pH modifiers such as citric acid, sodium citrate, succinic acid, phosphoric acid, sodium hydroxide and sodium carbonate; skin bleaching and lightening agents such as hydroquinone, kojic acid, ascorbic acid, magnesium ascorbyl phosphate and ascorbyl glucosamine; skin soothing and/or healing agents such as panthenol and derivatives like ethyl panthenol, aloe vera, pantothenic acid and its derivatives, allantoin; bisabolol and dipotassium glycyrrhizinate; retinoids such as retinol palmitate); tocopheryl nicotinate; skin treating agents; vitamins and derivatives thereof; and other similar materials.

Humectants have been described as agents that control the moisture exchange between the product and air, both in the container and on the skin. Humectants have also been described as compounds that prevent drying of skin or that increase the water content of the top layers of skin (e.g., hygroscopic compounds).

Suitable moisturizing agents include hydrophobic agents, hydrophilic agents and combinations thereof. Examples of moisturizing agents are allantoin, glycerol, polyglycerylmethacrylate, panthenol, polyols, ceramide, borage oil (linoleic acid), tocopherol (Vitamin E), tocopherol linoleate, dimethicone, hyaluronic acid, sodium peroxylinecarbolic acid (sodium PCA), wheat protein (e.g., laurdimonium hydroxypropyl hydrolyzed wheat protein), hair keratin amino acids, panthenol; primrose oil; GLA 3 and other fish oils that may include, for example, the omega-3 and omega-6 oils and/or linoleic acid; and flax seed oil, and mixtures thereof. Other moisturizing agents can also be used.

Numerous sunscreen agents are suitable for use in the personal care compositions of the present invention. Examples include, p-aminobenzoic acid, its salts and its derivatives, anthranilates, salicylates, cinnamic acid derivatives, dihydroxy cinnamic acid derivatives, trihydroxy cinnamic acid derivatives, dibenzalacetone, dibenzalacetophenone, naphtholsulfonates, dihydroxynaphtholic acid and its salts, coumarin derivatives, diazoles, quinine salts, quinoline derivatives, hydroxy- and methoxy-substituted benzophenones, uric and vilouric acids, tannic acid and its derivatives, hydroquinone and benzophenones. In accordance with this invention, an effective amount will normally be from about 0.01 to about 10% by weight, more particularly from about 0.1 to about 5% by weight, based on the weight of the composition.

Typically, the active ingredient in deodorant-antiperspirant compositions is a basic aluminum compound. Examples of such materials are aluminum chlorhydroxide, basic aluminum bromide, iodide or nitrate, and basic aluminum hydroxy chloride-zirconyl hydroxy oxychloride.

Cleaning agents are typically anionic, cationic, non-ionic or amphoteric surfactants. Typical anionic surfactants are carboxylates, sulfonates, sulfates or phosphates, e.g. fatty acid soaps, salts of lauryl sulfate and salts of lauryl ether sulfate. Examples of cationic surfactants are aliphatic mono, di and polyamines derived from fatty and rosin acids, amine oxides, ethoxylated alkyl amines and imidazolines. Examples of non-ionic surfactants are polyoxyethylene surfactants, alkylphenol ethoxylates, carboxylic acid esters, e.g., mono and diglycerides, polyoxyethylene esters and fatty acid diethanolamine condensates. Amphoteric surfactants are those containing combinations of the anionic and cationic groups described above, particularly those containing both acid carboxyls and basic nitrogen groups. Typical amphoteric surfactants are imidazolines and betaines, e.g., lauric and myristic imidazolines and betaines, and amidopropylbetaines.

A variety of medicinal agents also may be present as active ingredients in the compositions of the invention. Non-limiting examples are anti-acne additives, anti-cellulite agents, antihistamines, anti-inflammatory agents, antimicrobials, spermicides, antiseptics, antifungal agents and antiviral agents, and local anesthetics.

Examples of cosmetically or pharmaceutically acceptable carriers include, but are not limited to, a cream, a lotion, an emulsion, an oil, a spray, a gel, a aerosol, an aqueous or hydra-alcoholic solution, a suspension, a powder, a serums, an ointment, a shampoo, a gel, a paste and a mousse.

The composition may also include one or more adjuvants such as co-solvents, (e.g., alcohol, acetone, propylene carbonates), a diluent, a lubricant, an associative thickener, a surfactant, a flowing agent, an emollient, humectants, an antioxidant, a preservative, an antimicrobial agent, an antibiotic and an emulsifier.

The thickening additive compositions in accordance with this disclosure can be easily prepared by conventional methods known to persons of ordinary skill in the art, such as, simple mixing or blending using physical means or heat blending.

In one aspect, where acids and alcohols are included in the anhydrous solvent system, the acid or alcohol can be simply mixed with the moderately cross linked polyvinylpyrrolidone, using homogenizers at room temperature. This additive composition may be added to a base composition containing a personal care active and other adjuvants. This is particularly useful when the personal care composition is heat liable. The order of addition of the ingredients is not critical. In accordance with certain embodiments, the personal care active may be added to a dermatological or cosmetically acceptable carrier. Then, sufficient thickening additive composition may be added with stirring to provide a composition having the desired viscosity. Other optional adjuvants can then be added with continued stirring.

Where sunscreens and oils constitute at least a part of the anhydrous solvent system, heat can be applied, provided there is no detrimental effect on the personal care actives.

Sunscreen or combination of sunscreens when used in association with the thickening additive composition have shown viscosity as well stability excellence.

The thickening additive composition is highly effective for low pH personal care compositions. It is particularly useful for building viscosity in relatively acidic compositions, especially those containing C1-C25 alpha-or beta-hydroxycarboxylic acids.

Beyond building viscosity, the thickening system has the further advantage of stabilizing oil and water emulsions and providing a good skin-feel.

In another embodiment, oils containing surfactant were thickened with the thickening additive composition, which may also contain X-Tend™ 226 (ISP).

The following examples are presented to illustrate specific embodiments of the present compositions and methods.

### EXAMPLES

### Example 1: Thickening of various solvent systems and their rheological testing

Table 1 shows thickening of water, ethanol and combination of water and ethanol solvent system. The results were illustrated in Fig. 1 and 2. The hydroalcoholic solutions showed better results in terms of highly thickened additive composition have been obtained with 3% of lightly to moderately crosslinked polyvinylpyrrolidone solution. Still better results were obtained with 5% solution.

**Table 1:**

| **Solvent** | **Lightly to moderately crosslinked polyvinylpyr rolidone (%)** | **Deionized Water (%)** | **Ethanol v** | **Viscosity(TE @10RPM) (cP)** | **pH** |
|---|---|---|---|---|---|
| Water | 5 | 95 | - | 33,000 | 4.58 |
| Water | 3 | 97 | - | 7,000 | 4.67 |
| Ethanol | 5 | 95 | - | 40,000 | - |
| Ethanol | 3 | 97 | - | 7,500 | - |
| Water / Ethanol (50/50) | 5 | 47.50 | 47.50 | 41,000 | - |
| Water / Ethanol (50/50) | 3 | 48.50 | 48.50 | 9,500 | - |

### Example 2

Table 2 shows Compatibility testing of the various alcohol and water based gels in the presence of acids and their homologues bases/salts; solvent=water, light-to-moderately crosslinked PVP = 5%.

**Table 2:**

| **Active** | **%wt/wt Active** | **COA** |
|---|---|---|
| **None** | **-** | **Hazy thick gel** |
| **DHA** | **10** | **Hazy thick gel** |
| **DHA** | **5** | **Hazy thick gel** |
| **Glycolic acid** | **10** | **Hazy less viscous gel** |
| **Ascorbic acid** | **10** | **Hazy gel** |
| **Styleze W20** | **7** | **Hazy gel** |
| **Varisoft 300 CETAC** | **6** | **Hazy gel** |
| **DHA pH 3.5** | **10** | **Hazy thick gel** |

### Examples 3-7: Thickening Of Acidic Systems With Lightly To Moderately Crosslinked Polyvinylpyrrolidone

The low pH compositions, according to the present disclosure are made by mixing thickening agent i.e. strongly swellable, lightly to moderately crosslinked polyvinylpyrrolidone with alcohol/Deionized water/mixture of the two. This is followed by adding to it the various acidic components, such as salicylic acid and glycolic acid, with continuous homogenization. The viscosity of the resulting composition is measured using a Brookfield LVT viscometer.

The low pH compositions expressed as percents of total weight of the entire composition are reported in Table 3, and the corresponding results in Table 4.

**Table 3: Low pH Compositions:**

| | | |
|---|---|---|
| Example 3 | Ingredients | % w/w |
| | Lightly to moderately crosslinked polyvinylpyrrolidone | 6.00 |
| | Salicylic acid, USP | 10.00 |
| | SD alcohol 40 | 84.00 |
| | | |
| Example-4 | Lightly to moderately crosslinked polyvinylpyrrolidone | 4.50 |
| | Glycolic acid, (70% solution) | 43.00 |
| | Deionized Water | 52.50 |
| | | |
| Example-5 | Lightly to moderately crosslinked polyvinylpyrrolidone | 4.50 |
| | Glycolic acid, (70% solution) | 71.00 |
| | Deionized Water | 24.50 |
| | | |
| Example 6 | Lightly to moderately crosslinked polyvinylpyrrolidone | 4.50 |
| | Glycolic acid, (70% solution) | 71.00 |
| | Deionized Water | 14.50 |
| | SD alcohol 40 | 10.00 |
| | | |
| Example 7 | Lightly to moderately crosslinked polyvinylpyrrolidone | 4.50 |
| | Glycolic acid, (70% solution) | 71.00 |
| | Deionized Water | 4.50 |
| | SD alcohol 40 | 20.00 |

**Table 4: Results:**

| **Measurable Factors** | **Example - 3** | **Example-4** | **Example-5** | **Example-6** | **Example-7** |
|---|---|---|---|---|---|
| Appearance | Clear Gel | Translucent Gel | Translucent Gel | Translucent Gel | Translucent Gel |
| pH at 25°C | 2.9 | 1.68 | 1.32 | 1.35 | 1.45 |
| Viscosity at 25°C, Brookfield MTV, Spindle TC 10 rpm | 22,000 | 15,000 | 30,000 | 35,000 | 37,000 |

### Example 8-11: Thickening Of Alcohols Based Anhydrous Sunscreen With Lightly To Moderately Crosslinked Polyvinylpyrrolidone

The personal care compositions, which are substantially anhydrous, according to the present disclosure may be made by mixing thickening agent i.e. strongly swellable, lightly to moderately crosslinked polyvinylpyrrolidone with one or more substantially anhydrous solvent systems followed by adding it to the various personal care compositions. The viscosity of the resulting composition is measured using a Brookfield LVT viscometer.

The sunscreen compositions expressed as percents of total weight of the entire composition are reported in Table 5, and the corresponding results in Table 6.

**Table 5: Sunscreen Compositions:**

| | | |
|---|---|---|
| Example 8 | Ingredients | % w/w |
| | Lightly to moderately crosslinked polyvinylpyrrolidone | 3.00 |
| | Escalol 557 (O/M) | 7.50 |
| | Escalol 587(O/S) | 5.00 |
| | Escalol 597(Octocrylene) | 10.00 |
| | Escalol 567(Oxybenzone) | 6.00 |
| | SD alcohol 40 | 68.50 |
| | | |
| Example-9 | **Phase A** | |
| | Lightly to moderately crosslinked polyvinylpyrrolidone | 4.00 |
| | Escalol 517 (Avobenzone) | 3.00 |
| | Escalol 567(Oxybenzone) | 6.00 |
| | Escalol 587(Octisalate) | 5.00 |
| | Escalol 597(Octocrylene) | 6.00 |
| | Homosalate | 10.00 |
| | **Phase B** | |
| | SD alcohol 40 | 58.00 |
| | X-Tend™ 226 | 8.00 |
| | | |
| Example-10 | Lightly to moderately crosslinked polyvinylpyrrolidone | 5.00 |
| | Escalol 557 (O/M) | 7.50 |
| | Escalol 587(O/S) | 5.00 |
| | Escalol 517(A/B) | 3.00 |
| | Escalol 597(Octocrylene) | 10.00 |
| | Alcohol | 69.50 |
| | | |
| Example-11 | **Phase A** | |
| | Lightly to moderately crosslinked polyvinylpyrrolidone | 3.50 |
| | Escalol 517 (Avobenzone) | 3.00 |
| | Escalol 567(Oxybenzone) | 6.00 |
| | Escalol 587(Octisalate) | 5.00 |
| | Escalol 597(Octocrylene) | 6.00 |
| | Homosalate | 10.00 |
| | **Phase B** | |
| | SD alcohol 40 | 61.40 |
| | Vanilla Fragrance | 0.10 |

**Table 6: Results:**

| **Measurable Factors** | **Example -8** | **Example-9** | **Example-10** | **Example-11** |
|---|---|---|---|---|
| Appearance | Hazy Loose Gel | Hazy | Gelatinous, More Clear | Yellow Green Hazy Gel |
| pH | 7.03 | - | 6.86 | 5.87 |
| Viscosity (RVT/TD/10 RPM) | 1000 | 35,000 | 110,000 | 15,400 |
| SPF | - | - | - | 40-45 |
| Water Resistance Value | - | - | - | 97.64 % |

In Examples 8-10, thickening agent is first dispersed in alcohol and then added to a hot mixture of sunscreen oils. The mixture was continuously mixed until smooth and free of lumps and a gel was obtained. Compositions with the concentration of 5% lightly to moderately crosslinked polyvinylpyrrolidone (Example 3) exhibited better gel consistency and a viscosity of 110,000 cP.

In Example 11, thickening agent is first dispersed in sunscreen oils with heating and homogenization. The gel so obtained was cooled to 45°C. This is followed by addition of alcohol and fragrance with continuous mixing and the temperature was maintained at 30°C. The composition has shown high SPF value of 45 (on Labspere using 25 mg) or SPF of 41.4 (on Optimetrics SPF 290 Analyzer using 25 mg). The in-vitro water resistance value obtained was 97.64 (Std. Deviation of 6.12).

### Example 12:

| Formula name VOC Free SPF Gel | |
|---|---|
| **NB# 11810-99** | |
| | |
| **Ingredients** | **% W/W** |
| Ceraphyl 230 (Diisopropyl Adipate) | 19.50% |
| Ceraphyl 41 (C12-15 Alkyl Lactate) | 12.00% |
| Ceraphyl 368 (Octyl Palmitate) | 12.00% |
| Extend 226 (Phenylethyl Benzoate) | 9.00% |
| Escalol 517 (Avobenzone) | 3.00% |
| Escalol 567 (Benzophenone-3) | 6.00% |
| Escalol 587 (Octisalate) | 5.00% |
| Escalol 597 (Octocrylene) | 10.00% |
| NeoHeliopan HMS (Homosalate) | 15.00% |
| Si-Tec CM 40 | 5.00% |
| ACP 1120 lot 11755-49 | 3.50% |
| | 100.00% |

### Example 13:

| | 99/6 |
|---|---|
| **Ingredients** | **% W/W** |
| Ceraphyl 230 (Diisopropyl Adipate) | 29.50% |
| Ceraphyl 41 (C12-15 Alkyl Lactate) | 12.00% |
| Ceraphyl 368 (Octyl Palmitate) | 12.00% |
| Extend 226 (Phenylethyl Benzoate) | 9.00% |
| Escalol 517 (Avobenzone) | 3.00% |
| Escalol 567 (Benzophenone-3) | 6.00% |
| Escalol 587 (Octisalate) | 5.00% |
| Escalol 597 (Octocrylene) | |
| NeoHeliopan HMS (Homosalate) | 15.00% |
| Si-Tec CM 40 | 5.00% |
| ACP 1120 lot 11755-49 | 3.50% |
| | 100.00% |

### Example 14:

**Anhydrous VOC- Free**

| **High SPF Gel** | | |
|---|---|---|
| **With experimental Polymer ACP 1120** | | |
| **11691-120B** | | |
| **Ingredients** | **% W/W** | **Supplier** |
| **Phase A** | | |
| (Diisopropyl Adipate) **Ceraphyl®230** | 21.00 | **ISP** |
| (C12-15 Alkyl Lactate) **Ceraphyl®41** | 11.0 | **ISP** |
| (Ethylhexyl Palmitate) **Ceraphy® 368** | 11.0 | **ISP** |
| (Phenethyl Benzoate) **Extend 226** | 8.90 | **ISP** |
| (Avobenzone or Butyl Methoxydibenzoylmethane) **Escalol®517** | 3.00 | **ISP** |
| (Benzophenone-3 or Oxybenzone) **Escalol® 567** | 6.00 | **ISP** |
| (Octisalate or Ethylhexyl Salicylate) **Escalol®587** | 5.00 | **ISP** |
| (Octocrylene) **Escalol ®597** | 10.00 | **ISP** |
| (Homosalate) NeoHeliopan HMS | 15.00 | Symrise |
| | | |

| **Phase B** | | |
|---|---|---|
| (PVP/lightly crosslinked polymer) **ACP1120** | 4.00 | **ISP** |

| **Phase C** | | |
|---|---|---|
| (Cyclopentasiloxane) **Si-Tec™ CM 40** | 5.00 | **ISP** |
| Vanilla fragrance 854426 | 0.10 | Drom |
| **Total** | **100.00** | |

### Procedure

1. Weigh Phase A into a beaker.
2. Mix and heat until dissolved.
3. Cool to 40°C, add Phase B, use homogenizer or serrated blade.
4. Cool to 35°C, add Phase C.

**Typical Properties**

| | |
|---|---|
| Appearance | Yellow-green colored, clear gel |
| Viscosity | 49,000 cps (RVT/TD/10 RPM) |

Example 14 provides a broad spectrum, high SPF gel which is very water resistant, and has good rub resistance. The composition is a non alcohol formulation for non VOC regulations. It is non-greasy, with minimal shine.

## Claims

1. A sunscreen composition comprising a thickening additive composition wherein the thickening additive composition comprises: (A) one or more substantially anhydrous solvent systems comprising 15% or less water, the solvent including one or more of esters, glycols, acids, oils, emulsions and hydrocarbons; and (B) a thickening agent, wherein the thickening agent comprises a strongly swellable cross-linked polyvinylpyrrolidone having an aqueous gel volume of 15 to 150 mL/gm of polymer and a Brookfield viscosity in 5% aqueous solution of at least 10,000 cP at 25°C, measured using a Brookfield viscometer; wherein said thickening agent is from 1% to 6% by weight of said thickening additive composition.

2. A sunscreen composition according to claim 1 further comprising: (i) a personal care active and (ii) a cosmetically or dermatologically-acceptable carrier.

3. The sunscreen composition according to claim 1 or 2, wherein:
(a) said glycols are selected from the group consisting of propylene glycol, butylene glycol, dïethylenε glycol, dipropylene glycol, polyethylene glycol, and glycerine; and/or
(b) said oils are selected from the group consisting of castor bean oil, castor oil, rapeseed oil, soybean oil, palm kernel oil, babassu kernel oil, coconut oil, and mixtures; and/or
(c) said acids are selected from the group consisting of alpha and beta hydroxy acids, alpha hydroxyethanoic acid, alpha hydroxyoctanoic acid alpha hydroxycaprylic acid, ascorbic acid, adipic acid, citric acid, caprylic acid, capric acid, glycolic acid, lactic acid, lauric acid, malic acid, mixed fruit acids, myristic acid, palmitic acid, salicylic acid, stearic acid, linoleic acid, linolenic acid, ricinoleic acid, oleic acid, elaidic acid, erucic acid and mixtures thereof.

4. The sunscreen composition according to any one of claims 1 to 3, wherein said solvent system further comprises other chemicals selected from the group consisting of salts, electrolytes, acids, bases, surfactants, cations, di-hydroxyacetone and mixtures thereof.

5. The sunscreen composition according to claim 1 or claim 2, wherein said personal care composition is formulated in the form of liquid, gel, semi solid, or solid.

6. The sunscreen composition according to claim 2, wherein said cosmetically or pharmaceutically acceptable carrier is selected from the group consisting of a cream, a lotion, an emulsion, an oil, a spray, a gel, an aerosol, an aqueous or hydra-alcoholic solution, a suspension, a powder, a serum, an ointment, a shampoo, a gel, a paste and a mousse.

7. The sunscreen composition according to claim 2, wherein said thickening additive composition is from 0.5% to 10% by weight of said composition, optionally wherein said thickening additive composition is from 1% to 6% by weight of said composition.

8. The sunscreen composition according to claim 7, wherein said composition has viscosity of at least 10,000 cP at 3% concentration of thickening additive composition, optionally wherein said composition has viscosity of at least 30,000 cP at 5% concentration of thickening additive composition.

9. The sunscreen composition according to claim 2, wherein the composition further comprises at least one adjuvant selected from the group consisting of co-solvents, a diluent, a lubricant, an associative thickener, a surfactant, a flowing agent, an emollient, humectants, an antioxidant, a preservative, an antimicrobial agent, an antibiotic and an emulsifier.

## Patentansprüche

1. Sonnenschutz-Zusammensetzung, umfassend eine Verdickungsadditiv-Zusammensetzung, wobei die Verdickungsadditiv-Zusammensetzung Folgendes umfasst: (A) ein oder mehrere im Wesentlichen wasserfreie Lösungsmittelsystem, die 15 % oder weniger Wasser umfassen, wobei das Lösungsmittel eines oder mehrere von Estern, Glykolen, Säuren, Ölen, Emulsionen und Kohlenwasserstoffen umfasst; und (B) ein Verdickungsmittel, wobei das Verdickungsmittel ein stark quellbares vernetztes Polyvinylpyrrolidon umfasst, das als wässriges Gel ein Volumen von 15 bis 150 ml/g Polymer und bei 25 °C eine Brookfield-Viskosität in 5%iger wässriger Lösung von zumindest 10.000 cP, gemessen unter Verwendung eines Brookfield-Viskosimeters, aufweist; wobei das Verdickungsmittel 1 bis 6 Gew.-% der Verdickungsadditiv-Zusammensetzung ausmacht.

2. Sonnenschutz-Zusammensetzung nach Anspruch 1, die weiters Folgendes umfasst: (i) einen Körperpflegewirkstoff und (ii) einen kosmetisch oder dermatologisch annehmbaren Träger.

3. Sonnenschutz-Zusammensetzung nach Anspruch 1 oder 2, wobei:
(a) die Glykole aus der aus Propylenglykol, Butylenglykol, Diethylenglykol, Dipropylenglykol, Polyethylenglykol und Glycerin bestehenden Gruppe ausgewählt sind; und/oder
(b) die Öle aus der aus Rizinussamenöl, Rizinusöl, Rapsöl, Sojaöl, Palmkernöl, Babassukernöl, Kokosöl und Gemischen bestehenden Gruppe ausgewählt sind; und/oder
(c) die Säuren aus der aus α- und β-Hydroxysäuren, α-Hydroxyessigsäure, α-Hydroxyoctansäure, α-Hydroxycaprylsäure, Ascorbinsäure, Adipinsäure, Citronensäure, Caprylsäure, Caprinsäure, Glykolsäure, Milchsäure, Laurinsäure, Äpfelsäure, gemischten Fruchtsäuren, Myristinsäure, Palmitinsäure, Salicylsäure, Stearinsäure, Linolsäure, Linolensäure, Ricinolsäure, Ölsäure, Elaidinsäure, Erucasäure und Gemischen davon bestehenden Gruppe ausgewählt sind.

4. Sonnenschutz-Zusammensetzung nach einem der Ansprüche 1 bis 3, wobei das Lösungsmittelsystem weiters andere Chemikalien umfasst, die aus der aus Salzen, Elektrolyten, Säuren, Basen, Tensiden, Kationen, Dihydroxyaceton und Gemischen davon bestehenden Gruppe ausgewählt sind.

5. Sonnenschutz-Zusammensetzung nach Anspruch 1 oder 2, wobei die Körperpflegezusammensetzung in flüssiger, gelartiger, halbfester oder fester Form formuliert ist.

6. Sonnenschutz-Zusammensetzung nach Anspruch 2, wobei der kosmetisch oder pharmazeutisch annehmbare Träger aus der aus einer Creme, einer Lotion, einer Emulsion, einem Öl, einem Spray, einem Gel, einem Aerosol, einer wässrigen oder hydroalkoholischen Lösung, einer Suspension, einem Pulver, einem Serum, einer Salbe, einem Shampoo, einem Gel, einer Paste oder einem Mousse bestehenden Gruppe ausgewählt ist.

7. Sonnenschutz-Zusammensetzung nach Anspruch 2, wobei die Verdickungsadditiv-Zusammensetzung 0,5 bis 10 Gew.-% der Zusammensetzung ausmacht, wobei die Verdickungsadditiv-Zusammensetzung gegebenenfalls 1 bis 6 Gew.-% der Zusammensetzung ausmacht.

8. Sonnenschutz-Zusammensetzung nach Anspruch 7, wobei die Zusammensetzung bei einer Konzentration von 3 % der Verdickungsadditiv-Zusammensetzung eine Viskosität von zumindest 10.000 cP aufweist, wobei die Zusammensetzung gegebenenfalls bei einer Konzentration von 5 % der Verdickungsadditiv-Zusammensetzung eine Viskosität von zumindest 30.000 cP aufweist.

9. Sonnenschutz-Zusammensetzung nach Anspruch 2, wobei die Zusammensetzung weiters zumindest ein Adjuvans umfasst, das aus der aus Colösungsmitteln, einem Verdünner, einem Schmiermittel, einem assoziativen Verdickungsmittel, einem Tensid, einem Fließmittel, einem Weichmacher, Netzmitteln, einem Antioxidans, einem Konservierungsmittel, einem antimikrobiellen Mittel, einem Antibiotikum und einem Emulgator bestehenden Gruppe ausgewählt ist.

## Revendications

1. Composition d'écran solaire comprenant une composition d'additif épaississant, dans laquelle la composition d'additif épaississant comprend : (A) un ou plusieurs systèmes solvants pratiquement anhydres comprenant 15 % ou moins d'eau, le solvant comprenant un ou plusieurs parmi les esters, les glycols, les acides, les huiles, les émulsions et les hydrocarbures ; et (B) un agent épaississant, dans laquelle l'agent épaississant comprend une polyvinylpyrrolidone réticulée fortement gonflable ayant un volume de gel aqueux de 15 à 150 ml/g de polymère et une viscosité Brookfield en solution aqueuse à 5 % d'au moins 10 000 cP à 25°C, mesurée par utilisation d'un viscosimètre Brookfield ; et dans laquelle ledit agent épaississant représente de 1 % à 6 % en poids de ladite composition d'additif épaississant.

2. Composition d'écran solaire selon la revendication 1, comprenant en outre : (i) un principe actif de soin personnel et (ii) un véhicule acceptable du point de vue cosmétique ou dermatologique.

3. Composition d'écran solaire selon la revendication 1 ou 2, dans laquelle :
(a) lesdits glycols sont choisis dans l'ensemble constitué par le propylèneglycol, le butylèneglycol, le diéthylèneglycol, le dipropylèneglycol, le polyéthylèneglycol, et le glycérol ; et/ou
(b) lesdites huiles sont choisies dans l'ensemble constitué par l'huile de graines de ricin, l'huile de ricin, l'huile de colza, l'huile de soja, l'huile de palmiste, l'huile de babassu, l'huile de coprah, et leurs mélanges ; et/ou
(c) lesdits acides sont choisis dans l'ensemble constitué par les hydroxyacides alpha et bêta, l'acide alpha-hydroxyéthanoïque, l'acide alpha-hydroxyoctanoïque, l'acide alpha-hydroxycaprylique, l'acide ascorbique, l'acide adipique, l'acide citrique, l'acide caprylique, l'acide caprique, l'acide glycolique, l'acide lactique, l'acide laurique, l'acide malique, les acides de fruits mixtes, l'acide myristique, l'acide palmitique, l'acide salicylique, l'acide stéarique, l'acide linoléique, l'acide linolénique, l'acide ricinoléique, l'acide oléique, l'acide élaïdique, l'acide érucique et leurs mélanges.

4. Composition d'écran solaire selon l'une quelconque des revendications 1 à 3, dans laquelle ledit système solvant comprend en outre d'autres produits chimiques choisis dans l'ensemble constitué par les sels, les électrolytes, les acides, les bases, les tensioactifs, les cations, la dihydroxyacétone et leurs mélanges.

5. Composition d'écran solaire selon la revendication 1 ou la revendication 2, dans laquelle ladite composition de soin personnel est formulée sous la forme d'un liquide, d'un gel, d'un semi-solide, ou d'un solide.

6. Composition d'écran solaire selon la revendication 2, dans laquelle ledit véhicule acceptable du point de vue cosmétique ou pharmaceutique est choisi dans l'ensemble constitué par une crème, une lotion, une émulsion, une huile, un spray, un gel, un aérosol, une solution aqueuse ou hydro-alcoolique, une suspension, une poudre, un sérum, une pommade, un shampooing, un gel, une pâte et une mousse.

7. Composition d'écran solaire selon la revendication 2, dans laquelle ladite composition d'additif épaississant représente de 0,5 % à 10 % en poids de ladite composition, éventuellement dans laquelle ladite composition d'additif épaississant représente de 1 % à 6 % en poids de ladite composition.

8. Composition d'écran solaire selon la revendication 7, laquelle composition a une viscosité d'au moins 10 000 cP à une concentration de 3 % de la composition d'additif épaississant, éventuellement laquelle composition a une viscosité d'au moins 30 000 cP à une concentration de 5 % de la composition d'additif épaississant.

9. Composition d'écran solaire selon la revendication 2, laquelle composition comprend en outre au moins un adjuvant choisi dans l'ensemble constitué par les co-solvants, un diluant, un lubrifiant, un épaississant associatif, un tensioactif, un agent de fluidité, un émollient, les humectants, un antioxydant, un conservateur, un agent antimicrobien, un antibiotique et un émulsionnant.
